# FASCICULE DE BREVET EUROPEEN

(11) **EP 2 783 207 B1**
(45) Date de publication et mention de la délivrance du brevet: **03.02.2016**
(21) Numéro de dépôt: 12808426.6
(22) Date de dépôt: 21.11.2012
(51) Int. Cl.: G01N 29/44, B21J 5/00, B22D 46/00, G01N 21/95, G06T 7/00, G01N 29/04

(54) **SYSTÈME ET PROCÉDÉ DE LOCALISATION DYNAMIQUE D'UN DÉFAUT CONSTATÉ SUR UNE PIÈCE**
SYSTEM UND VERFAHREN ZUR DYNAMISCHEN LOKALISIERUNG EINES DETEKTIERTEN DEFEKTS IN EINEM WERKSTÜCK
SYSTEM AND METHOD FOR LOCALISING DYNAMICALLY A DETECTED FLAW IN A WORKPIECE

(30) Priorité: 23.11.2011 FR 1160699
(43) Date de publication de la demande: 01.10.2014
(73) Titulaire: SNECMA, 75015 Paris (FR)
(72) Inventeur: LEBLANC, Jonathan, F-77550 Moissy-Cramayel Cedex (FR)
(74) Mandataire: Ahner, Philippe
(86) Numéro de dépôt international: PCT/FR2012/052682
(87) Numéro de publication internationale: WO 2013/076421

(56) Documents cités:
- US-A- 5 337 611
- US-B1- 6 776 212
- US-B1- 7 092 484

## Description

### DOMAINE TECHNIQUE

La présente invention se rapporte au domaine général de forgeage et concerne la localisation dynamique d'un défaut constaté sur une pièce défectueuse en relation avec l'opération de forgeage. Elle trouve une application dans tous les domaines industriels et en particulier le domaine de l'aéronautique dans lequel les pièces forgées sont soumises à des contraintes de qualité et de sûreté maximales.

### ÉTAT DE LA TECHNIQUE ANTÉRIEURE

Lors de la mise en forme de pièces forgées, des aléas de production peuvent conduire à la création de défauts entraînant potentiellement le rebut de ces pièces. La détection de défauts sur les pièces forgées peut être réalisée par des moyens de contrôle de type ultrasonore, magnétoscopique, ou visuel.

L'origine d'un défaut peut être liée à des paramètres de forgeage ou à un défaut de configuration des outils de mise en forme. Ainsi, l'identification ou la localisation de l'origine d'un défaut est importante pour améliorer l'opération ou les outils de forgeage.

Actuellement, pour identifier l'origine d'un défaut sur une pièce forgée, on rapporte de manière approximative le défaut sur la pièce d'origine avant forgeage. Ce report de défauts est imprécis et est réalisé uniquement pour des défauts présents en surface et dans des zones facilement identifiables visuellement.

En outre, avec ce type de report, on ne peut pas localiser un défaut généré lors d'une étape intermédiaire de forgeage et on ne peut pas analyser la propagation du défaut, ce qui est préjudiciable à un bon diagnostic du défaut.

Le document US 7,092,484 divulgue un système de localisation dynamique d'un défaut constaté sur une pièce défectueuse par modélisant un procès dynamique d'une pièce pour obtenir un modèle de la pièce et du défaut.

Ainsi, l'objet de la présente invention est de proposer un système et un procédé de localisation dynamique d'un défaut constaté sur une pièce défectueuse remédiant aux inconvénients précités et permettant de connaître de manière prospective ou rétrospective la propagation du défaut.

### EXPOSÉ DE L'INVENTION

La présente invention est définie par un système de localisation dynamique d'un défaut constaté sur une pièce défectueuse en relation avec une opération de forgeage, comportant:
- des moyens de traitement pour modéliser une opération de mise en forme par forgeage d'une pièce selon un ensemble de modèles successifs de ladite pièce,
- des moyens de traitement pour ajouter à un premier modèle dudit ensemble de modèles un traceur de défaut sur une zone correspondant à la région du défaut de ladite pièce défectueuse pour former un premier modèle tracé, et
- des moyens de traitement pour suivre temporellement ledit traceur de défaut lors de ladite modélisation à partir dudit premier modèle tracé afin de localiser l'origine dudit défaut.

Ceci permet de diagnostiquer prospectivement ou rétrospectivement la propagation du défaut dans le milieu de la pièce.

Avantageusement, la zone associée audit traceur de défaut dans le premier modèle présente des dimensions et un emplacement sensiblement similaires aux dimensions et emplacement de la région du défaut sur la pièce défectueuse.

Selon un mode de réalisation particulier de la présente invention, la modélisation de l'opération de mise en forme est une modélisation dynamique par éléments finis formant à chaque étape courante de modélisation un maillage polygonal représentatif de la pièce à l'étape de forgeage correspondante.

Avantageusement, les moyens de traitement sont configurés pour définir à chaque étape courante de modélisation, les dimensions et emplacement dudit traceur de défaut en fonction des éléments élémentaires dudit maillage à ladite étape courante.

Selon un premier mode de réalisation de la présente invention, ledit ensemble de modèles successifs comprend un modèle initial correspondant à la pièce avant forgeage, des modèles intermédiaires correspondant à des étapes de forgeage intermédiaires, et un modèle final correspondant à la pièce forgée, ledit premier modèle correspondant audit modèle final et le premier modèle tracé correspondant à un modèle final tracé, les moyens de traitement étant configurés pour suivre temporellement ledit traceur de défaut en inversant le déroulement de ladite modélisation à partir dudit modèle final tracé.

Les moyens de traitement sont configurés pour localiser ledit traceur de défaut sur le modèle initial afin d'identifier la zone où le défaut aurait été sur la pièce avant forgeage.

Avantageusement, les moyens de traitement sont configurés pour localiser ledit traceur de défaut sur des modèles intermédiaires comportant des configurations particulières pour vérifier si lesdites configurations particulières sont susceptibles d'induire ledit défaut.

Selon un deuxième mode de réalisation de la présente invention, ledit ensemble de modèles successifs comprend un modèle initial correspondant à la pièce avant forgeage et un modèle final correspondant à la pièce forgée, ledit premier modèle correspondant audit modèle initial et ledit premier modèle tracé correspondant à un modèle initial tracé, les moyens de traitement étant configurés pour localiser ledit traceur de défaut sur le modèle final afin de vérifier si le défaut sur la pièce forgée est inscrit en dehors d'une zone d'usinage de ladite pièce forgée.

Avantageusement, ledit traceur de défaut est un élément de contraste associé audit maillage polygonal.

L'invention vise également un procédé de localisation dynamique d'un défaut constaté sur une pièce défectueuse en relation avec une opération de forgeage, comportant les étapes suivantes :
- modéliser une opération de mise en forme par forgeage d'une pièce selon un ensemble de modèles successifs de ladite pièce,
- ajouter à un premier modèle dudit ensemble de modèles un traceur de défaut sur une zone correspondant à la région du défaut de ladite pièce défectueuse pour former un premier modèle tracé, et
- suivre temporellement ledit traceur de défaut lors de ladite modélisation à partir dudit premier modèle tracé afin de localiser l'origine dudit défaut.

### BRÈVE DESCRIPTION DES DESSINS

On décrira à présent, à titre d'exemples non limitatifs, des modes de réalisation de l'invention, en se référant aux dessins annexés, dans lesquels :
La Fig. 1 illustre de manière schématique un procédé de forgeage ;
La Fig. 2 illustre de manière schématique un système 12 de localisation dynamique d'un défaut constaté sur une pièce 1 défectueuse, selon l'invention ;
La Fig. 3 illustre de manière schématique un ensemble de modèles successifs de la mise en forme de la pièce, selon l'invention ;
Les Figs. 4 et 4A-4D illustrent un procédé de localisation dynamique d'un défaut constaté sur une pièce défectueuse, selon un mode de réalisation préféré de l'invention ;
Les Figs. 5A-5E illustrent une modélisation de forgeage avec un lopin désaxé par rapport aux outillages ; et
Les Figs. 6 et 6A-6C illustrent un procédé de localisation dynamique d'un défaut constaté sur une pièce défectueuse, selon un autre mode de réalisation de l'invention.

### EXPOSÉ DÉTAILLÉ DES MODES DE RÉALISATION PRÉFÉRÉS

Le concept à la base de l'invention est d'utiliser la modélisation de la mise en forme de la pièce pour suivre la propagation du défaut.

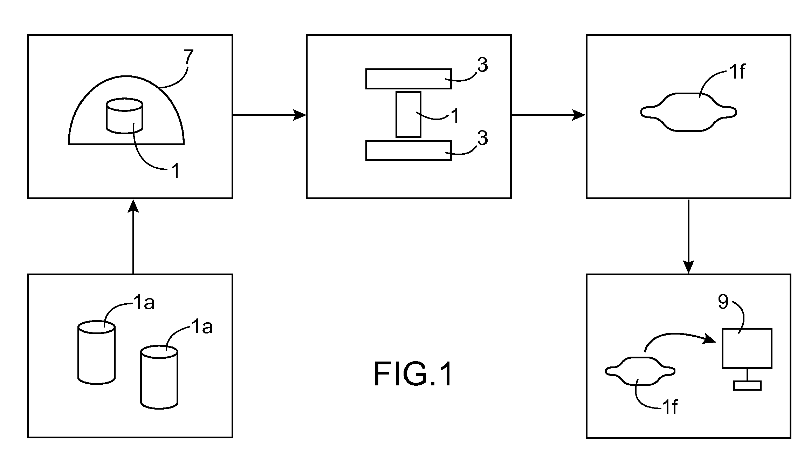

La Fig. 1 illustre de manière schématique un procédé de forgeage.

Les pièces à forger qui sont en général de formes cylindriques appelés lingots ou lopins 1a sont déformées à froid ou à chaud entre deux matrices ou outils de forgeage 3 par martelage ou par pression à l'aide d'une presse de forge, pour former au final des pièces forgées. La pièce finale 1f ressemble souvent à un gros disc ou une assiette avec une géométrie particulière.

Pour la déformation à chaud, la pièce 1 est mise dans un four 7 avant l'étape d'écrasement et dans certains cas, les étapes de forgeage et d'écrasement sont répétées plusieurs fois avant d'obtenir la pièce finale 1f.

A la fin du forgeage, on réalise un contrôle en utilisant par exemple, des moyens 9 ultrasonore, magnétoscopique, ou visuel afin de vérifier qu'une pièce forgée 1f ne comporte pas de défauts avant par exemple de commencer une opération d'usinage sur cette pièce 1f.

La présente invention propose de localiser le défaut de manière dynamique afin d'identifier l'origine de ce défaut. Ceci permet par exemple de savoir si le défaut est isolé ou s'il est reproductible sur une série de pièces et dans ce cas de mener des actions correctives pour éviter la reproduction de ce défaut.

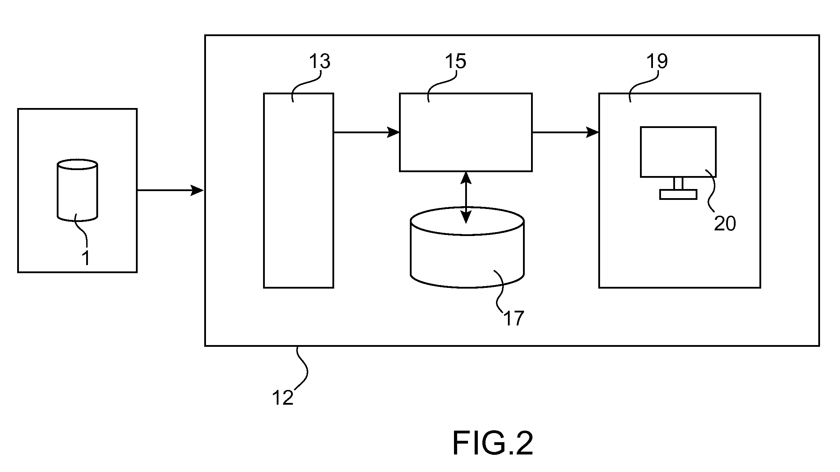

En effet, la Fig. 2 illustre de manière schématique un système 12 de localisation dynamique d'un défaut constaté sur une pièce 1 défectueuse, selon l'invention.

Par localisation dynamique d'un défaut, on entend le repérage spatiotemporel du défaut relativement à l'espace occupé par la pièce 1 et aux moments successifs de sa mise en forme. Autrement dit, c'est la localisation spatiale du défaut dans la pièce 1 à chaque moment de sa transformation.

Le système 12 de localisation comporte des moyens d'entrée 13 de données, des moyens de traitement 15, des moyens de stockage 17 et des moyens de sortie 19 comprenant des moyens de visualisation 20. Les moyens de traitement 15 permettent d'exécuter un ou plusieurs programmes d'ordinateur comprenant des instructions de code de programme, stockés dans les moyens de stockage 17 et conçus pour mettre en oeuvre le procédé de localisation dynamique d'un défaut.

Plus particulièrement, les moyens de traitement 15 sont configurés pour modéliser l'opération de mise en forme par forgeage d'une pièce 1 selon un ensemble de modèles successifs de la pièce.

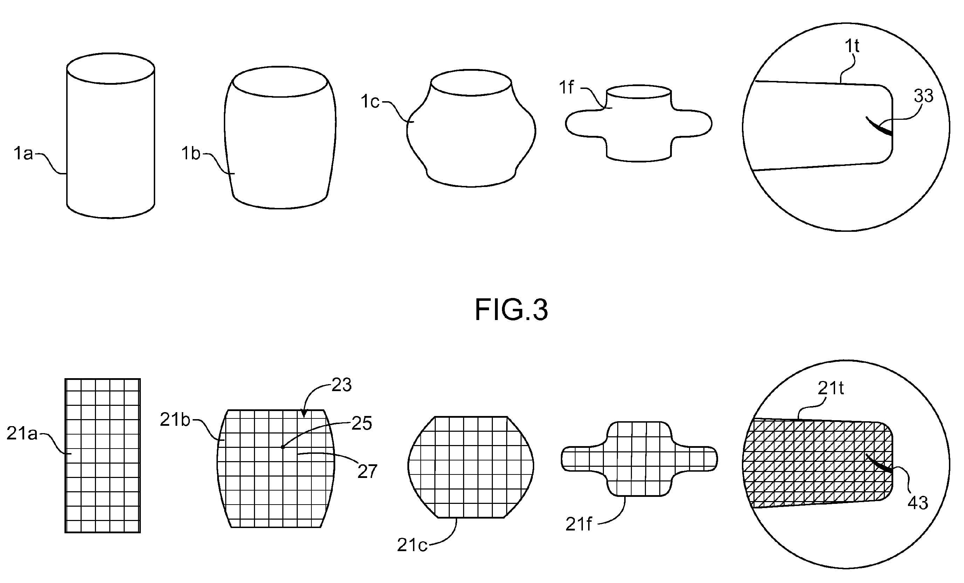

En effet, la Fig. 3 illustre de manière schématique un ensemble de modèles 21a-21t successifs de la mise en forme de la pièce 1 comportant un modèle initial 21a représentatif du lopin 1a (c'est-à-dire, la pièce avant forgeage), des modèles intermédiaires 21b, 21c représentatifs des pièces intermédiaires de forgeage 1b, 1c, et un modèle final 21f représentatif de la pièce forgée 1f (c'est-à-dire, la pièce finale après forgeage).

On notera que la modélisation peut être réalisée en trois dimensions (3D) ou éventuellement en deux dimensions (2D) pour des pièces axisymétriques.

Ainsi, les moyens de traitement 15 sont utilisés pour résoudre numériquement des équations modélisant la déformation de la pièce 1 sous l'action des outils de forgeage 3 en fonction des paramètres de forgeage comportant par exemple, un champ de température de la pièce 1 et des outils 3, un champ de pression, des coefficients d'échange thermique, la masse volumique de la pièce 1, un champ de vitesse de déformation, etc. Ces paramètres permettent à la modélisation numérique d'être le plus possible représentative des opérations réelles de forgeage en atelier. Par exemple, les coefficients d'échange thermique permettent de tenir compte lors de l'opération de forgeage, de la chaleur dissipée par la pièce 1 dans le milieu environnant par rayonnement et/ou convection et en particulier lorsque la température de la pièce 1 est élevée (par exemple, de l'ordre de 1000°C).

La résolution numérique est effectuée de manière itérative et utilise par exemple des maillages 23 pour discrétiser le domaine géométrique continu de la pièce par des éléments finis décrits par des sommets ou noeuds 25 et des arêtes 27. Ainsi, la modélisation dynamique par éléments finis forme à chaque itération ou étape courante de modélisation, un maillage polygonal 23 (par exemple triangulaire) représentatif de la pièce 1 à l'étape de forgeage correspondante.

Lorsqu'un défaut 33 est constaté sur une pièce défectueuse 1t, on réalise un relevé comportant des données relatives au défaut 33 (par exemple, les dimensions et la position du défaut sur la pièce 1t) et on importe ces données dans le système 12 de localisation dynamique.

En effet, on utilise les moyens d'entrée 13 du système 12 de localisation dynamique pour introduire les données relatives au défaut 33 permettant aux moyens de traitement 15 d'insérer un équivalent du défaut 33 dans le modèle 21t correspondant à la pièce défectueuse 1t.

Plus particulièrement, les moyens de traitement 15 sont configurés pour ajouter à un premier modèle 21t appartenant à l'ensemble de modèles 21a-21t de l'opération de forgeage, un traceur de défaut 43 sur une zone correspondant à la région du défaut de la pièce 1t défectueuse pour former un premier modèle tracé 21t. Autrement dit, le premier modèle tracé 21t représente la pièce défectueuse 1t au moment de la constatation du défaut.

Avantageusement, la zone associée au traceur de défaut 43 dans le premier modèle 21t présente des dimensions et un emplacement sensiblement similaires aux dimensions et emplacement de la région du défaut 33 sur la pièce défectueuse 1t.

Les moyens de traitement 15 en relation avec les moyens de visualisation 20 permettent ensuite de suivre temporellement le traceur de défaut 43 lors de la modélisation à partir du premier modèle tracé 21t pour diagnostiquer la dynamique du défaut 33. Ainsi, la propagation du défaut 33 dans la pièce 1 peut être suivie de manière prospective (c'est-à-dire, dans le sens du temps) ou rétrospective (c'est-à-dire, dans le sens inverse du temps) à partir du premier modèle tracé 21t.

En particulier, à chaque étape courante de modélisation, les dimensions et emplacement du traceur de défaut 43 peuvent être définis en fonction des éléments élémentaires (c'est-à-dire, noeuds 25 et/ou arêtes 27) du maillage 23 à l'étape courante.

A titre d'exemple, le traceur de défaut 43 est un élément de contraste qui représente le défaut 33 en termes de dimensions et de position et qui peut être intégré au maillage polygonal 23 du modèle 21 correspondant par une technique connue de type CAO. Par exemple, le traceur de défaut 43 peut être représenté par un contour de couleur contrastée par rapport au maillage 23, enfermant une surface sensiblement égale à celle du défaut réel 33, et défini en fonction des noeuds 25 voisins du défaut. On notera qu'on n'a pas besoin de connaître ni la nature ni la forme précise du défaut 33.

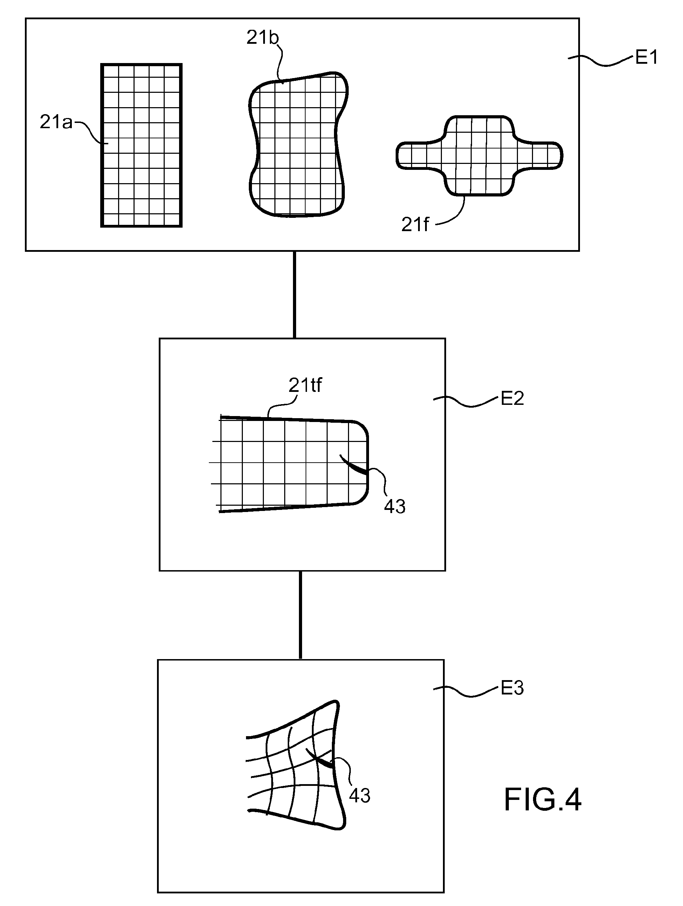

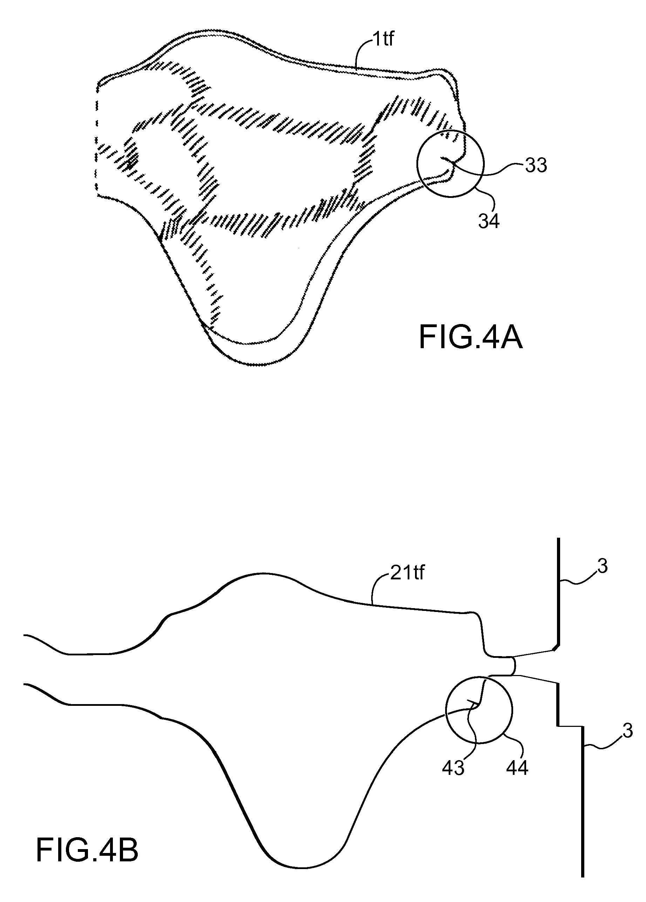

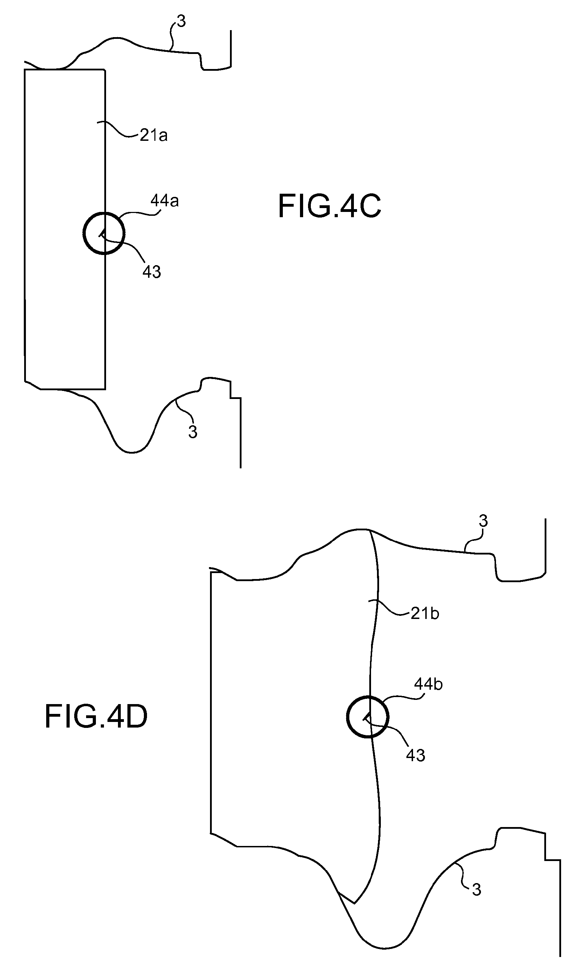

Les Figs. 4 et 4A-4D illustrent un procédé de localisation dynamique d'un défaut constaté sur une pièce défectueuse, selon un mode de réalisation préféré de l'invention.

Selon ce mode de réalisation, le premier modèle auquel le traceur de défaut est ajouté correspond au modèle final 21f représentatif de la pièce forgée 1f de sorte que le premier modèle tracé 21t correspond à un modèle tracé final 21tf représentatif d'une pièce forgée défectueuse 1tf.

En effet, à l'étape E1, les moyens de traitement 15 modélisent l'opération de mise en forme par forgeage d'une pièce 1 selon des modèles 21a-21f successifs comprenant un modèle initial 21a correspondant à la pièce avant forgeage (ou lopin) 1a et un modèle final 21f correspondant à la pièce forgée 1f.

A l'étape E2, après qu'un défaut a été constaté sur une pièce forgée défectueuse (voir Fig. 4A), les moyens de traitement 15 ajoutent au modèle final 21f un traceur de défaut 43 sur une zone correspondant à la région du défaut de la pièce forgée 1f défectueuse pour former un modèle tracé final 21tf (voir Fig. 4B).

En effet, la Fig. 4A est un exemple illustrant une partie ou plus précisément la moitié d'une pièce forgée 1tf défectueuse présentant un repli de forge 33 (par exemple, une petite fissure) sur une coupe micrographique de la pièce 1. Par ailleurs, la Fig. 4B illustre en 2D la partie correspondante du modèle tracé final 21tf intégrant le traceur de défaut 43 sur une zone 44 correspondante à la région 34 du défaut de la pièce forgée défectueuse 1tf de la Fig. 4A.

A l'étape E3, les moyens de traitement 15 en relation avec les moyens de visualisation 20 vont permettre de suivre temporellement le traceur de défaut 43 en inversant la cinématique (c'est-à-dire, en inversant le déroulement de la modélisation), à partir du modèle tracé final 21tf pour identifier l'origine du défaut de la pièce forgée 1tf défectueuse (voir Figs. 4C et 4D). Ceci permet de retracer l'historique de la région qui a subit le défaut.

En effet, au cours de la modélisation de forgeage, le maillage polygonal 23 du modèle 21 représentant la pièce 1 se déforme au cours du temps. Autrement dit, les positions relatives des noeuds 25 se modifient les uns par rapport aux autres au cours de la transformation. Ceci engendre des variations dans l'étendue et la localisation du traceur de défaut 43 qui est défini par rapport à des noeuds 25 voisins dont les coordonnées sont connues à chaque étape de la modélisation. Ainsi, en remontant le temps on peut identifier la morphologie, la géométrie et le positionnement du défaut 33 avant l'écrasement de la pièce 1.

On notera que le défaut 33 peut résulter d'un défaut originel de la matière du lopin 1a, ou d'un défaut lié aux paramètres de forgeage (vitesse de déformation, température, etc), ou d'un défaut géométrique de la pièce 1 et/ou des outils 3 de mise en forme.

La Fig. 4C illustre la localisation du traceur de défaut 43 sur le modèle initial 21a. Ceci permet d'identifier la zone 44a où le défaut aurait été s'il était présent sur le lopin 1a.

Afin d'identifier d'autres sources du défaut, on utilise le système 12 de localisation dynamique pour localiser le traceur de défaut 43 sur des modèles intermédiaires susceptibles d'induire le défaut.

A titre d'exemple, la Fig. 4D illustre la localisation du traceur de défaut 43 sur un modèle intermédiaire 21b comportant une configuration particulière.

Ce modèle intermédiaire 21b montre que le traceur de défaut 43 est positionné au voisinage d'une zone 44b de concavité normalement générée par la pénétration des outillages 3 dans la matière de la pièce 1 à forger.

Ainsi, une concavité ou une inflexion anormalement prononcée dans cette zone 44b peut être responsable du repli ou défaut 33 dans la pièce défectueuse 1tf. On notera qu'une accentuation de la concavité peut par exemple résulter d'un défaut de parallélisme entre les matrices ou outils 3 de forgeage et/ou d'un mauvais centrage du lopin 1a par rapport au centre des outillages 3.

Il est alors intéressant de modéliser les différents scénarios qui génèrent une accentuation de concavité afin de diagnostiquer ou de vérifier si c'est bien la concavité qui a conduit à ce défaut 33.

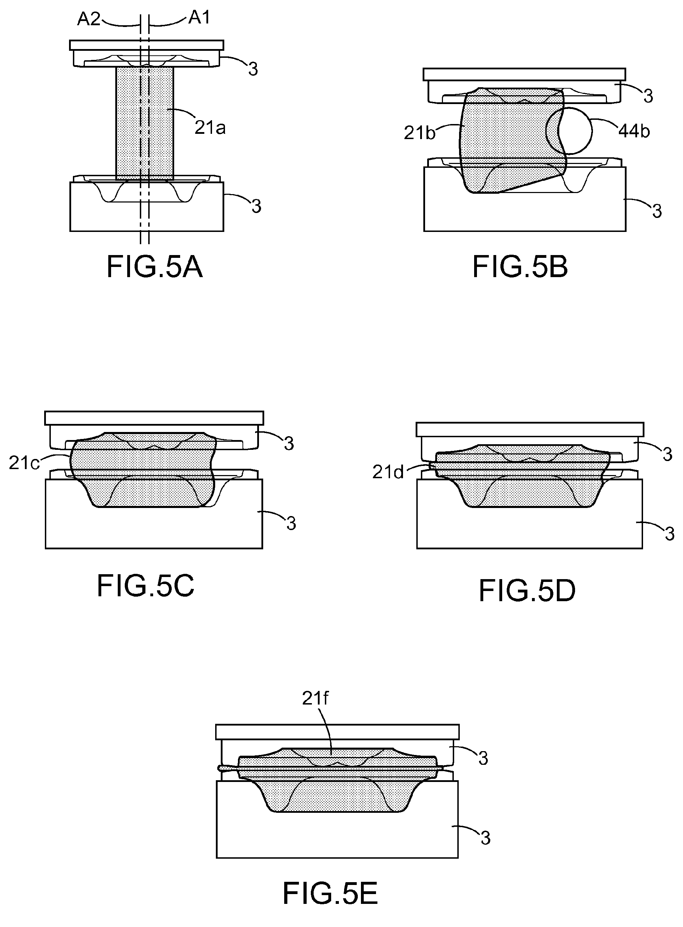

A titre d'exemple, les Figs. 5A-5E illustrent une modélisation de forgeage avec un lopin 21a délibérément désaxé par rapport aux outillages 3.

La Fig. 5A montre un lopin 21a dont l'axe central A1 est désaxé de quelques millimètres par rapport à l'axe de symétrie A2 des outillages 3.

La Fig. 5B montre clairement une augmentation de la concavité dans la zone 44b de concavité originelle au voisinage de laquelle a été localisé le défaut. Ceci met clairement en évidence un fort risque de repli dans la zone de concavité comme illustré sur les Figs 5C-5E. En particulier, la Fig. 5E montre que le modèle final 21f de la pièce forgée est très dissymétrique.

Ainsi, on peut modifier la configuration ou la géométrie des outils 3 de forgeage pour que la pénétration des deux noyaux des outillages 3 dans la pièce 1 ne génère plus de concavité ou pour que l'écoulement de la matière se fasse différemment afin que la concavité soit réduite ou supprimée.

D'une manière générale, la localisation de l'origine du défaut 33 selon l'invention permet de modéliser les différents effets ou scénarios qui peuvent conduire au défaut afin d'y remédier.

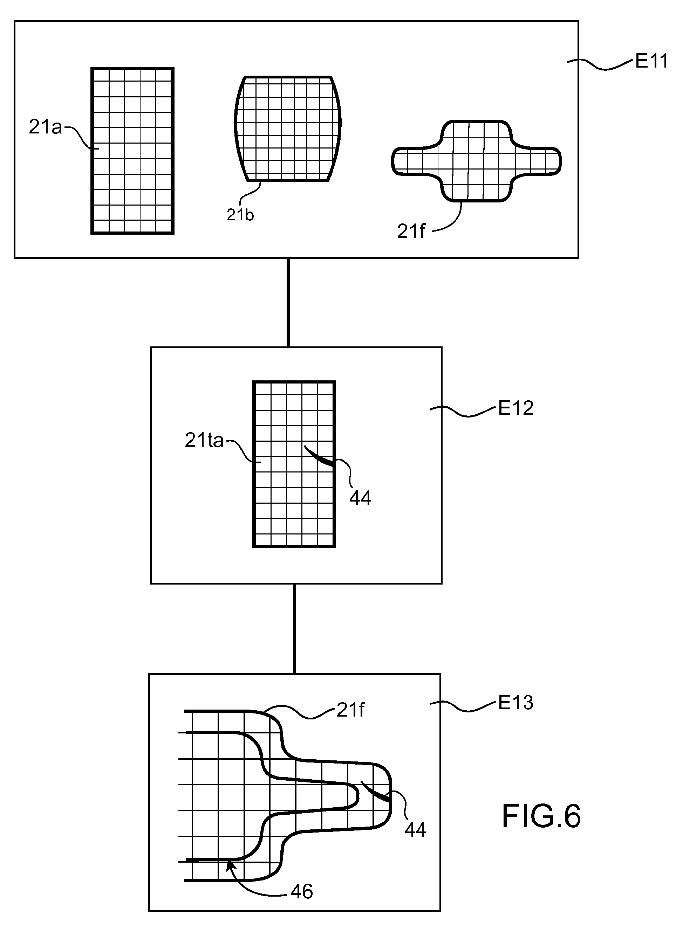

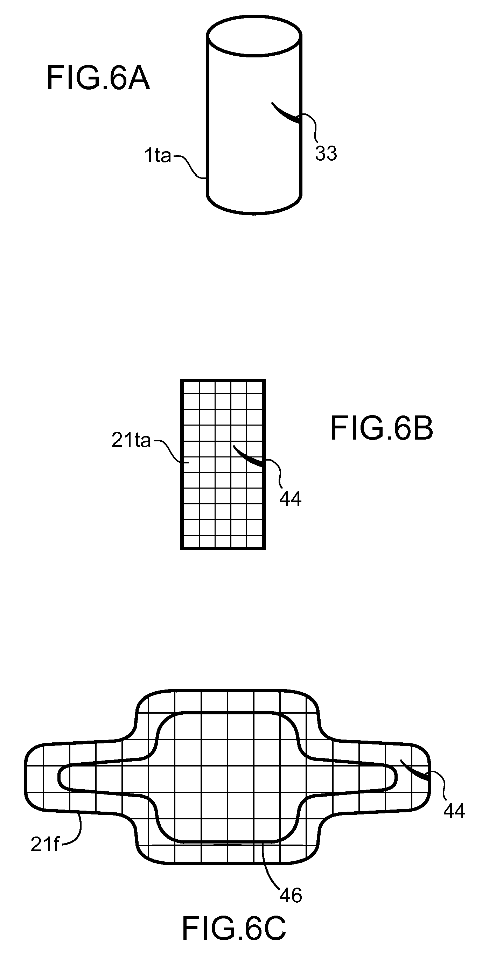

Les Fig. 6 et 6A-6C illustrent un procédé de localisation dynamique d'un défaut constaté sur une pièce défectueuse, selon un autre mode de réalisation de l'invention.

Selon ce mode de réalisation, le premier modèle auquel le traceur de défaut 44 est ajouté correspond au modèle initial 21a représentatif de la pièce avant forgeage (le lopin) 1a de sorte que le premier modèle tracé correspond à un modèle tracé initial 21ta représentatif d'un lopin défectueux 1ta.

A l'étape E11, les moyens de traitement 15 modélisent l'opération de mise en forme par forgeage d'une pièce selon des modèles successifs comprenant un modèle initial 21a correspondant à la pièce 1a avant forgeage et un modèle final 21f correspondant à la pièce forgée 1f.

A l'étape E12, après qu'un défaut 33 ait été constaté sur un lopin défectueux 1ta (voir Fig. 6A), les moyens de traitement 15 ajoutent au modèle initial 21a un traceur de défaut 44 sur une zone correspondant à la région du défaut 33 du lopin défectueux 1ta pour former un modèle tracé initial 21ta (voir Fig. 6B).

En effet, la Fig. 6A est un exemple illustrant un lopin défectueux 1ta présentant un petit défaut 33 sur sa surface et la Fig. 6B illustre en 2D le modèle tracé initial 21ta intégrant le traceur de défaut 44 sur une zone correspondante à la région du défaut 33 du lopin défectueux 1ta de la Fig. 6A.

A l'étape E13, les moyens de traitement 15 en relation avec les moyens de visualisation 20 vont permettre de localiser le traceur de défaut 44 sur le modèle final 21f afin de vérifier si le défaut sur la pièce forgée est inscrit en dehors d'une zone d'usinage 46 de cette pièce forgée. Ceci permet d'économiser des pièces en vérifiant si la pièce forgée après usinage sera impactée ou non par le défaut 33 initial sur la pièce 1ta avant forgeage.

La Fig. 6C illustre la localisation du traceur de défaut 44 sur le modèle final ainsi que le contour d'usinage 46. Cet exemple montre que le défaut ne touche pas la pièce finie après usinage et par conséquent, le lopin de départ peut être utilisé.

## Revendications

1. Système de localisation dynamique d'un défaut (33) constaté sur une pièce obtenue à la suite d'une opération de forgeage d'un lopin, **caractérisé en ce qu'**il comporte :
- des moyens de traitement (15) adaptés à modéliser ladite opération de forgeage par la résolution numérique d'équations modélisant la déformation de la pièce (1) durant ladite opération de forgeage en fonction de paramètres de forgeage, ladite résolution étant effectuée de manière itérative et aboutissant à un ensemble de modèles (21a-21f) temporellement successifs de la mise en forme de ladite pièce (1) durant ladite opération de forgeage, ledit ensemble comprenant un modèle initial (21a) correspondant audit lopin (1a), des modèles intermédiaires (21b-21e) correspondant à des étapes de forgeage intermédiaires et un modèle final (21f) correspondant à ladite pièce (1) forgée,
- des moyens d'entrées pour fournir audit moyen de traitement (15), des données relatives à un défaut (33) constaté sur ladite pièce (1), une pièce présentant un défaut étant dite pièce défectueuse (1t),
- des moyens de traitement (15) adaptés à ajouter à un premier modèle appartenant audit ensemble de modèles (21a-21f), un traceur de défaut (43) sur une zone correspondant à la région du défaut de ladite pièce défectueuse (1t) pour former un premier modèle tracé (21t) représentant la pièce défectueuse (1t) au moment de la constatation du défaut, et
- des moyens de visualisation (20) en relation avec des moyens de traitement (15), adaptés à suivre temporellement ledit traceur de défaut (43) lors de ladite modélisation à partir dudit premier modèle tracé (21t), de manière rétrospective ou prospective, afin de diagnostiquer la dynamique dudit défaut (33).

2. Système selon la revendication 1, **caractérisé en ce que** la zone associée audit traceur de défaut (43) dans le premier modèle tracé (21t) présente des dimensions et un emplacement sensiblement similaires aux dimensions et emplacement de la région du défaut (33) sur la pièce défectueuse (1t).

3. Système selon la revendication 1 ou 2, **caractérisé en ce que** la modélisation de ladite opération de forgeage est une modélisation dynamique par éléments finis formant à chaque itération, un maillage polygonal (23) représentatif de la pièce à l'étape de forgeage correspondante.

4. Système selon la revendication 3, **caractérisé en ce que** les moyens de traitement (15) sont configurés pour définir à chaque itération, les dimensions et emplacement dudit traceur de défaut (43) en fonction des éléments élémentaires dudit maillage polygonal (23) à ladite étape courante.

5. Système selon l'une quelconque des revendications 1 à 4, **caractérisé en ce que** ledit premier modèle correspond audit modèle final (21f) et le premier modèle tracé (21t) correspond à un modèle tracé final (21tf), et **en ce que** les moyens de traitement (15) sont configurés pour suivre temporellement ledit traceur de défaut (43) en inversant le déroulement de ladite modélisation à partir dudit modèle tracé final (21tf).

6. Système selon la revendication 5, **caractérisé en ce que** les moyens de traitement (15) sont configurés pour localiser ledit traceur de défaut (43) sur le modèle initial (21a) afin d'identifier la zone où le défaut aurait été sur la pièce avant forgeage.

7. Système selon la revendication 5 ou 6, **caractérisé en ce que** les moyens de traitement (15) sont configurés pour localiser ledit traceur de défaut (43) sur des modèles intermédiaires comportant des configurations particulières pour vérifier si lesdites configurations particulières sont susceptibles d'induire ledit défaut.

8. Système selon l'une quelconque des revendications 1 à 4, **caractérisé en ce que** ledit premier modèle correspond audit modèle initial (21a) et ledit premier modèle tracé (21t) correspond à un modèle tracé initial (21ta), et **en ce que** les moyens de traitement (15) sont configurés pour localiser ledit traceur de défaut (43) sur le modèle final (21f) afin de vérifier si le défaut sur la pièce forgée est inscrit en dehors d'une zone d'usinage (46) de ladite pièce forgée.

9. Système selon l'une quelconque des revendications 3 à 8, **caractérisé en ce que** ledit traceur de défaut (43) est un élément de contraste associé audit maillage polygonal (23).

10. Procédé de localisation dynamique d'un défaut (33) constaté sur une pièce obtenue à la suite d'une opération de forgeage d'un lopin, **caractérisé en ce qu'**il comporte les étapes suivantes :
- modéliser ladite opération de forgeage par la résolution numérique d'équations modélisant la déformation de la pièce (1) durant ladite opération de forgeage en fonction de paramètres de forgeage, ladite résolution étant effectuée de manière itérative et aboutissant à un ensemble de modèles (21a-21f) temporellement successifs de la mise en forme de ladite pièce (1) durant l'opération de forgeage, ledit ensemble comprenant un modèle initial (21a) correspondant audit lopin (1a), des modèles intermédiaires (21b-21e) correspondant à des étapes de forgeage intermédiaires et un modèle final (21f) correspondant à ladite pièce (1) forgée,
- obtenir des données relatives à un défaut (33) constaté sur ladite pièce (1), une pièce présentant un défaut étant dite pièce défectueuse (1t),
- ajouter à un premier modèle appartenant audit ensemble de modèles un traceur de défaut (43) sur une zone correspondant à la région du défaut de ladite pièce défectueuse pour former un premier modèle tracé (1t) représentant la pièce défectueuse (1t) au moment de la constatation du défaut, et
- suivre temporellement ledit traceur de défaut (43) lors de ladite modélisation à partir dudit premier modèle tracé, de manière rétrospective ou prospective, afin de diagnostiquer la dynamique afin de diagnostiquer la dynamique dudit défaut.

## Patentansprüche

1. System zur dynamischen Lokalisierung eines Fehlers (33), der an einem Werkstück festgestellt wird, welches in Folge einer Operation des Schmiedens eines Rohlings erhalten wird, **dadurch gekennzeichnet, dass** es umfasst:
- Verarbeitungsmittel (15), die dazu ausgelegt sind, die Schmiedeoperation zu modellieren durch numerisches Lösen von Gleichungen, die die Verformung des Werkstücks (1) während der Schmiedeoperation als Funktion von Schmiedeparametern modellieren, wobei das Lösen in iterativer Weise ausgeführt wird und zu einem Ensemble von zeitlich aufeinanderfolgenden Modellen (21a - 21f) der Formung des Werkstücks (1) während der Schmiedeoperation führt, wobei das Ensemble ein Anfangsmodell (21a) umfasst, welches dem Rohling (1a) entspricht, Zwischenmodelle (21b - 21e), die Zwischenschmiedeschritten entsprechen, sowie ein Endmodell (21f), welches dem geschmiedeten Werkstück (1) entspricht,
- Eingabemittel, um dem Verarbeitungsmittel (15) Daten bezüglich eines Fehlers (33) zu liefern, der an dem Werkstück (1) festgestellt wird, wobei ein Werkstück, das einen Fehler aufweist, als fehlerhaftes Werkstück (1t) bezeichnet wird,
- Verarbeitungsmittel (15), die dazu ausgelegt sind, zu einem ersten Modell, das zu dem Ensemble von Modellen (21a - 21f) gehört, einen Fehlermarkierer (43) bei einer Zone hinzufügen, die der Region des Fehlers des fehlerhaften Werkstücks (1t) entspricht, um ein erstes markiertes Modell (21t) zu bilden, das das fehlerhafte Werkstück (1t) im Zeitpunkt der Feststellung des Fehlers darstellt, und
- Visualisierungsmittel (20) in Verbindung mit Verarbeitungsmitteln (15), die dazu ausgelegt sind, dem Fehlermarkierer (43) während der Modellierung ausgehend von dem ersten markierten Modell (21t) zeitlich in rückschauender oder vorrausschauender Weise zu folgen, um die Dynamik des Fehlers (33) zu diagnostizieren.

2. System nach Anspruch 1, **dadurch gekennzeichnet, dass** die Zone, die dem Fehlermarkierer (43) in dem ersten markierten Modell (21t) zugeordnet ist, Abmessungen und eine Platzierung im Wesentlichen ähnlich zu den Abmessungen und der Platzierung der Region des Fehlers (33) bei dem fehlerhaften Werkstück (1t) aufweist.

3. System nach Anspruch 1 oder 2, **dadurch gekennzeichnet, dass** die Modellierung der Schmiedeoperation eine dynamische Modellierung durch finite Elemente ist, die bei jeder Iteration eine polygonale Vernetzung (23) bildet, welche repräsentativ ist für das Werkstück im entsprechenden Schmiedeschritt.

4. System nach Anspruch 3, **dadurch gekennzeichnet, dass** die Verarbeitungsmittel (15) dazu ausgelegt sind, bei jeder Iteration die Abmessungen und die Platzierung des Fehlermarkierers (43) als Funktion der elementaren Elemente der polygonalen Vernetzung (23) beim laufenden Schritt zu definieren.

5. System nach einem der Ansprüche 1 bis 4, **dadurch gekennzeichnet, dass** das erste Modell dem Endmodell (21f) entspricht, und das erste markierte Model (21t) einem markierten Endmodell (21tf) entspricht, und dass die Verarbeitungsmittel (15) dazu ausgelegt sind, zeitlich dem Fehlermarkierer (43) durch Invertieren des Ablaufs der Modellierung ausgehend von dem markierten Endmodell (21tf) zu folgen.

6. System nach Anspruch 5, **dadurch gekennzeichnet, dass** die Verarbeitungsmittel (15) dazu ausgelegt sind, den Fehlermarkierer (43) bei dem Anfangsmodell (21a) zu lokalisieren, um die Zone zu identifizieren, wo der Fehler bei dem Werkstück vor dem Schmieden gewesen wäre.

7. System nach Anspruch 5 oder 6, **dadurch gekennzeichnet, dass** die Verarbeitungsmittel (15) dazu ausgelegt sind, den Fehlermarkierer (43) bei Zwischenmodellen zu lokalisieren, die besondere Konfigurationen umfassen, um zu überprüfen, ob die besonderen Konfigurationen geeignet sind, den Fehler auszulösen.

8. System nach einem der Ansprüche 1 bis 4, **dadurch gekennzeichnet, dass** das erste Modell dem Anfangsmodell (21a) entspricht, und das erste markierte Model (21t) einem markierten Anfangsmodell (21ta) entspricht, und dass die Verarbeitungsmittel (15) dazu ausgelegt sind, den Fehlermarkierer (43) bei dem Endmodell (21f) zu lokalisieren, um zu überprüfen, ob der Fehler bei dem geschmiedeten Werkstück außerhalb einer Bearbeitungszone (46) des geschmiedeten Werkstücks eingeschrieben ist.

9. System nach einem der Ansprüche 3 bis 8, **dadurch gekennzeichnet, dass** der Fehlermarkierer (43) ein Kontrastelement ist, welches der polygonalen Vernetzung (23) zugeordnet ist.

10. Verfahren zur dynamischen Lokalisierung eines Fehlers (33) der bei einem Werkstück festgestellt wird, das in Folge einer Operation des Schmiedens eines Rohlings erhalten wird, **dadurch gekennzeichnet, dass** es die folgenden Schritte umfasst:
- Modellieren der Schmiedeoperation durch numerisches Lösen von Gleichungen, die die Verformung des Werkstücks (1) während der Schmiedeoperation als Funktion von Schmiedeparametern modellieren, wobei das Lösen in iterativer Weise ausgeführt wird und zu einem Ensemble von zeitlich aufeinanderfolgenden Modellen (21a - 21f) der Formung des Werkstücks (1) während der Schmiedeoperation führt, wobei das Ensemble ein Anfangsmodell (21a) umfasst, das dem Rohling (1a) entspricht, Zwischenmodelle (21b-21e), die Zwischenschmiedeschritten entsprechen, sowie ein Endmodell (21f), das dem geschmiedeten Werkstück (1) entspricht,
- Erhalten von Daten betreffend einen Fehler (33), der bei dem Werkstück (1) festgestellt wird, wobei ein Werkstück, das einen Fehler aufweist, als fehlerhaftes Werkstück (1t) bezeichnet wird,
- Hinzufügen, zu einem ersten Modell, das zu dem Ensemble von Modellen gehört, eines Fehlermarkierers (43) in einer Zone, die der Region des Fehlers des fehlerhaften Werkstücks entspricht, um ein erstes markiertes Modell (1t) zu bilden, das das fehlerhafte Werkstück (1t) im Zeitpunkt der Feststellung des Fehlers darstellt, und
- zeitliches Verfolgen des Fehlermarkierers (43) während der Modellierung ausgehend von dem ersten markierten Modell in zurückschauender oder vorrausschauender Weise, um die Dynamik zu des Fehlers (33) zu diagnostizieren.

## Claims

1. A system for the dynamic locating of a fault (33) observed in a component obtained after a forging operation of a blank, **characterized in that** it comprises:
- processing means (15) adapted for the modelling of said forging operation via the numerical solving of equations modelling working of the component (1) during the said forging operation as a function of forging parameters, the said solving being performed iteratively and leading to a set of time successive models (21a-21f) of the forming of said component (1) during the said forging operation, the said set comprising an initial model (21a) corresponding to the said blank (1a), intermediate models (21b-21e) corresponding to intermediate forging steps and a final model (21f) corresponding to the said forged component (1);
- input means to provide the said processing means (15) with data relating to a fault (33) observed in said component (1), a component having a fault being called a defective component (1t);
- processing means (15) adapted to add a fault plotter (43) to a first model belonging to the said set of models (21a-21f), in a zone corresponding to the region of the fault in the said defective component (1t) to obtain a first plotted model (21t) representing the defective component (1t) at the time of detection of the fault; and
- viewing means (20) linked with the processing means (15) and adapted to track the said fault plotter (43) over time during the said modelling starting from the said first plotted model (21t), retrospectively or prospectively, to diagnose the dynamics of the said fault (33).

2. The system according to claim 1, **characterized in that** the dimensions and positioning of the zone associated with the said fault plotter (43) in the first plotted model (21t) are substantially similar to the dimensions and positioning of the region of the fault (33) in the defective component (1t).

3. The system according to claim 1 or 2, **characterized in that** the modelling of the said forging operation is dynamic modelling with finite elements forming a polygon mesh (23) at each iteration representing the component at the corresponding forging step.

4. The system according to claim 3, **characterized in that** the processing means (15) are configured to define at each iteration the dimensions and position of the said fault plotter (43) as a function of the elementary elements of the said polygon mesh (23) at the said step in time.

5. The system according to any of claims 1 to 4, **characterized in that** the said first model corresponds to the said final model (21f) and the first plotted model (21t) corresponds to a final plotted model (21tf), and **in that** the processing means (15) are configured to track in time the said fault plotter (43) by reversing the sequence of the said modelling starting from the said final plotted model (21tf).

6. The system according to claim 5, **characterized in that** the processing means (15) are configured to locate the said fault plotter (43) in the initial model (21a) to identify the region in which the fault would have been in the component before forging.

7. The system according to claim 5 or 6, **characterized in that** the processing means (15) are configured to locate the said fault plotter (43) in intermediate models comprising particular configurations to verify whether the said particular configurations are likely to cause the said fault.

8. The system according to any of claims 1 to 4, **characterized in that** the said first model corresponds to the said initial model (21a) and the said first plotted model (21t) corresponds to an initial plotted model (21ta), and **in that** the processing means (15) are configured to locate the said fault plotter (43) in the final model (21f) to verify whether the fault in the forged component lies outside a machining region (46) of the said forged component.

9. The system according to any of claims 3 to 8, **characterized in that** the said fault plotter (43) is a contrast element associated with the said polygon mesh (23).

10. A method for dynamically locating a fault (33) observed in a component obtained after the forging operation of a blank, **characterized in that** it comprises the following steps:
- modelling the said forging operation by the numerical solving of equations modelling the working of the component (1) during the said forging operation as a function of forging parameters, the said solving being performed iteratively and leading to a set of time successive models (21a-21f) of the forming of the said component (1) during the forging operation, the said set comprising an initial model (21a) corresponding to the said blank (1a), intermediate models (21b-21e) corresponding to intermediate forging steps and a final model (21f) corresponding to the said forged component (1);
- obtaining data relating to a fault (33) observed in said component (1), a component having a fault being called a defective component (1t);
- adding a fault plotter (43) to the first model belonging to said set of models, in a zone corresponding to the region of the fault in the said defective component to obtain a first plotted model (1t) representing the defective component (1t) at the time of detection of the fault; and
- tracking the said fault plotter (43) in time during the said modelling starting from the said first plotted model, retrospectively or prospectively, in order to diagnose the dynamics of the said fault.
